# EUROPEAN PATENT SPECIFICATION

(11) **EP 0 909 328 B1**
(45) Date of publication and mention of the grant of the patent: **29.09.2010**
(21) Application number: 96922632.3
(22) Date of filing: 01.07.1996
(51) Int. Cl.: C12P 1/00, C12P 7/06, C12P 7/08, C12P 7/54, C12P 7/62, C12P 21/04, C12N 1/20, C12P 1/04, C12P 7/02, C12P 7/40, C12P 21/00

(54) **BIOLOGICAL PRODUCTION OF ACETIC ACID FROM WASTE GASES**
BIOLOGISCHE HESTELLUNG VON ESSIGSäURE AUS ABGASEN
PRODUCTION BIOLOGIQUE D'ACIDE ACETIQUE A PARTIR DE GAZ RESIDUAIRES

(43) Date of publication of application: 21.04.1999
(73) Proprietor: Emmaus Foundation, Inc., Fayetteville, Arkansas 72701 (US)
(72) Inventor: GADDY, James, L., Fayetteville, AR 72703 (US); Chen, Guangjiong, Chesterfield, MO 63017 (US)
(74) Representative: Manaton, Ross Timothy
(86) International application number: PCT/US1996/011146
(87) International publication number: WO 1998/000558

(56) References cited:
- US-A- 5 077 208
- US-A- 5 173 429
- CHEMICAL ABSTRACTS, vol. 125, no. 2, 8 July 1996 (1996-07-08) Columbus, Ohio, US; abstract no. 15072, CLAUSEN, E. C. ET AL: "Ethanol from biomass by gasification/fermentation" XP002117032 & CONVERS. UTIL. WASTE MATER. ( 1996 ), 157-167. EDITOR(S): KHAN, M. RASHID. PUBLISHER: TAYLOR & FRANCIS, WASHINGTON, D. C. ,
- CHEMICAL ABSTRACTS, vol. 123, no. 1, 3 July 1995 (1995-07-03) Columbus, Ohio, US; abstract no. 7982, "High pressure synthesis gas conversion: Task 3: High pressure profiles" XP002117033 & REPORT ( 1993 ), DOE/PC/91028-T6;ORDER NO. DE93040517, 53 PP. AVAIL.: NTIS FROM: ENERGY RES. ABSTR. 1993, 18(11), ABSTR. NO. 32677,
- CHEMICAL ABSTRACTS, vol. 120, no. 22, 30 May 1994 (1994-05-30) Columbus, Ohio, US; abstract no. 275289, "Biological production of ethanol from coal: Task 4 report, continuous reactor studies" XP002117034 & REPORT ( 1992 ), DOE/PC/89876-T17;ORDER NO. DE93008947, 146 PP. AVAIL.: NTIS FROM: ENERGY RES. ABSTR. 1993, 18(5), ABSTR. NO. 11669,
- CHEMICAL ABSTRACTS, vol. 119, no. 25, 20 December 1993 (1993-12-20) Columbus, Ohio, US; abstract no. 269118, "High pressure synthesis gas fermentation, January 15, 1991-April 14, 1991" XP002117035 & REPORT ( 1991 ), DOE/PC/91028-T5;ORDER NO. DE92019656, 21 PP. AVAIL.: NTIS FROM: ENERGY RES. ABSTR. 1992, 17(12), ABSTR. NO. 33274,
- CHEMICAL ABSTRACTS, vol. 119, no. 7, 16 August 1993 (1993-08-16) Columbus, Ohio, US; abstract no. 67962, TANNER, RALPH S. ET AL: "Clostridium ljungdahlii sp. nov., an acetogenic species in clostridial rRNA homology group I" XP002117036 & INT. J. SYST. BACTERIOL. ( 1993 ), 43(2), 232-6 ,
- CHEMICAL ABSTRACTS, vol. 117, no. 14, 5 October 1992 (1992-10-05) Columbus, Ohio, US; abstract no. 134106, KLASSON, K. T. ET AL: "Bioliquefaction of coal synthesis gas" XP002117037 & PREPR. PAP. - AM. CHEM. SOC., DIV. FUEL CHEM. ( 1992 ), 37(4), 1977-82 ,
- CHEMICAL ABSTRACTS, vol. 115, no. 9, 2 September 1991 (1991-09-02) Columbus, Ohio, US; abstract no. 90621, ELMORE, BILL BAUCUM: "Biological production of ethanol from coal synthesis gas using Clostridium ljungdahlii, strain PETC" XP002117038 & ( 1990 ) 307 PP. AVAIL.: UNIV. MICROFILMS INT., ORDER NO. DA9111206 FROM: DISS. ABSTR. INT. B 1991, 51(11), 5459,
- INTERNATIONAL JOURNAL OF SYSTEMATIC BACTERIOLOGY, April 1979, Vol. 29, DASHEKVICZ et al., "Identification of a Carbon Monoxide-Metabolizing Bacterium as a Strain of Rhodopseudomonas Gelatinosa (Molisch) van Niel", pages 145-148, XP002941905.
- APPLIED BIOCHEMISTRY AND BIOTECHNOLOGY, 1988, Vol. 18, BARIK et al., "Biological Production of Alcohols from Coal Through Indirect Liquefaction", pages 363-378, XP002941906.
- APPLIED BIOCHEMISTRY AND BIOTECHNOLOGY, 1990, Vol. 24/25, KLASSON et al., "Biological Production of Liquid and Gaseous Fuels from Synthesis Gas", pages 857 - 873, XP002941908.
- APPLIED BIOCHEMISTRY AND BIOTECHNOLOGY, 1989, Vol. 20/21, VEGA et al., "The Biological Production of Ethanol from Synthesis Gas", pages 781-797, XP002941909.
- CRUEGER et al., "Biotechnology, A Textbook of Industrial Microbiology", SINAUER ASSOCIATED, INC., SUNDERLAND, MA, 1989, pages 74-89. XP008075556
- DEMAIN et al., Industrial Microbiology and Biotechnology, ASM, 1986, pages 332-335, XP002941972.
- PROC. NATL. ACAD. SCI. U.S.A., September 1976, Vol. 73, No. 9, UFFEN R.L., "Anaerobic Growth of Rhodopseudomonas Species in the Dark with Carbon Monoxide as Sole Carbon and Energy Substrate", pages 3298-3302, XP002941910.
- FEBS LETTERS, February 1983, Vol. 152, No. 1, STUPERICH et al., "Carbon Monoxide Fixation into the Carbonyl Groups of Acetyl Coenzyme A During Autotrophic Growth of Methanobacterium", pages 21-23, XP002941922
- JOURNAL OF BACTERIOLOGY, April 1984, Vol. 158, No. 1, O'BRIEN et al., "Association of Hydrogen Metabolism with Unitrophic or Mixotrophic Growth of Methanosarcina Barkeri on Carbon Monoxide", pages 373-375, XP002941911.
- JOURNAL OF BACTERIOLOGY, February 1983, Vol. 153, No. 2, MILLER et al., "Oxidation of Hydrogen and Reduction of Methanol to Methane is the Sole Energy Source for a Methanogen Isolated from Human Feces", pages 1051-1055, XP002941912.
- FEMS MICROBIOLOGY LETTERS, 1983, Vol. 17, DIEKERT et al., "Carbon Monoxide Fixation into the Carboxyl Group of Acetate During Growth of Acetobacterium Woodii on H2 and C02", pages 299-302, XP002941913.
- INTERNATIONAL JOURNAL OF SYSTEMATIC BACTERIOLOGY, January 1988, Vol. 38, No. 1, NAKAMURA et al., "Taxonomic Study of Bacillus Coagulans Hammer 1915 with a Proposal for Bacillus Smithii sp. Nov.", pages 63-73, XP002941907.
- ARCHIVES OF MICROBIOLOGY, November 1984, Volume 139, No. 4, KRUEGER et al., "Thermophilic Bacilli Growing with Carbon Monoxide", pages 402-408, XP002941944.

## Description

The general field of the invention relates to biological methods, processes, microorganisms, and apparatus for producing products, materials, intermediates, and the like such as organic acids, single cell protein ("SCP"), hydrogen, alcohols, and organic acid salts from the waste gas streams of certain industrial processes. More particularly, the invention concerns processes utilizing continuous gaseous substrate fermentation under anaerobic conditions to produce acetic acid and its salts, and to microorganisms useful in such processes.

The conventional procedure for producing organic acids, alcohols, hydrogen and organic acid salts is chemical synthesis of petroleum-derived feedstocks. The rapidly escalating cost of petroleum has generated considerable interest in producing these valuable commodities by fermentative processes that utilize renewable or waste materials as the feedstock. Single cell protein is produced as a by-product of the fermentations to be used as an animal feed supplement.

There is also growing concern over the massive amounts of atmospheric pollutants and greenhouse gases produced by conventional industrial processes. The Environmental Protection Agency recently estimated that over six million metric tons of carbon monoxide and nearly four million metric tons of hydrogen were discharged annually by the industrial complex. A substantial portion of this waste carbon monoxide and hydrogen is the result of carbon black manufacture and coke production, roughly 2.6 million metric tons of CO and 0.5 million metric tons of H₂. Large amounts of carbon monoxide or hydrogen are also produced by the ammonia industry (125, 144 metric tons of CO in 1991), petroleum refining (8 metric tons per thousand barrels), steel mills (152 pounds per metric ton of steel produced), and sulfate pulping of wood (286 pounds per ton of pulp). In 1991, the adipic acid industry generated 40,773 metric tons of carbon monoxide that was burned for fuel value or flared. In many cases, these gases are discharged directly to the atmosphere, placing a heavy pollution burden on the environment.

Typically, the waste gases from the manufacture of industrial products are released at low pressures and temperatures. Current technology cannot utilize these dilute gases under such conditions. Adapting existing technology to separate and recover hydrogen or carbon monoxide from these waste streams would be expensive and impractical.

In light of the foregoing, there is a need for a cost effective and practical method, microorganism, and apparatus for utilizing the above-described waste gases and for producing products, materials, intermediates and the like such as organic acids, alcohols, hydrogen and organic acid salts by other than chemical synthesis of petroleum derived feedstocks.

### SUMMARY OF THE INVENTION

In accordance with the present invention, acetic acid is produced from the waste carbon monoxide, hydrogen, and/or carbon dioxide of industrial processes, thereby reducing environmental pollution while at the same time saving energy and chemical feedstocks.

The present invention provides, in one aspect, a process for producing acetic acid, comprising:
(a) providing a continuous flow of gas comprising:
   (i) a gas comprising carbon monoxide;
   (ii) a gas comprising carbon monoxide and hydrogen; or
   (iii) a gas comprising hydrogen and carbon dioxide;
   into a bioreactor, said bioreactor containing an aqueous nutrient medium and the anaerobic bacterium *C. ljungdahlii* ERI-2, ATCC No. 55380;
(b) directing a flow of said aqueous nutrient medium into said bioreactor; and
(c) fermenting said gas and said nutrient medium using said bacterium at a low pH under conditions that permit converting the gas to said acetic acid in a broth.

The acetic acid is recovered from the aqueous phase in a separate vessel or vessels, utilizing a suitable recovery process. Examples of recovery processes include extraction, distillation or combinations thereof, or other efficient recovery processes. The bacteria are removed from the aqueous phase and recycled to avoid toxicity and maintain high cell concentrations, thus maximizing reaction rates. Cell separation, if desired, is accomplished by centrifugation, membranous ultrafiltration, or other techniques.

The invention also provides, in an alternative aspect, a biologically pure culture of the microorganism *Clostridium ljungdahlii* ERI-2 [ATCC No. 55380].

The principal object of the present invention is the provision of a process and/or microorganism for the production of acetic acid and its salts from carbon monoxide, hydrogen, and/or carbon dioxide. Another object of the present invention is the provision of methods, microorganisms and apparatus for the production of acetic acid and its salts from the waste gas streams of industrial processes such as oil refining, carbon black, coke, ammonia, and methanol production.

A still further object of the present invention is the provision of a process for producing acetic acid from a waste gas stream of identical composition to that found in the manufacture of carbon black.

Yet another and more particular object of the present invention is the provision of a method, microorganism and apparatus involving continuous gaseous substrate fermentation under anaerobic conditions to accomplish the conversion of waste gas streams of certain industrial processes into useful products, namely acetic acid and its salts.

Other objects and further scope of the applicability of the present invention will become apparent from the detailed description to follow, taken in conjunction with the accompanying drawings wherein like parts are designated by like reference numerals.

### Brief Description of the Drawings

Fig. 1 is a schematic diagram of a process for the production of acetic acid from waste gas.
Fig. 2 is a schematic diagram of a process for the production of CMA from waste gas.
Fig. 3 is a schematic representation of a continuous fermentation system in accordance with an embodiment of the present invention.
Fig. 4 is a graphical illustration of cell concentration (OD) versus time.
Fig. 5 is a graphical representation of acetic acid (HAC) versus time.

### Detailed Description of the Invention

The term "waste gas" or "waste gas streams" as used herein means carbon monoxide and hydrogen mixed with other elements or compounds, including carbon dioxide, nitrogen and methane, in a gaseous state and which are typically released or exhausted to the atmosphere either directly or through combustion. Normally, release takes place under standard smokestack temperatures and pressures. Accordingly, the processes of the present invention are suitable for converting these atmospheric pollutants into useful products, namely acetic acid plus salts, such as calcium magnesium acetate (CMA) and potassium acetate (KA).

Anaerobic bacteria which are known to convert carbon monoxide and water or hydrogen and carbon dioxide into alcohols and acids and acid salts include *Acetobacterium kivui, A. woodii, Clostridium aceticum, Butyribacterium methylotrophicum, C. acetobutylicum, C. formoaceticum, C. kluyveri, C. thermoaceticum, C. thermocellum, C. thermohydrosulfuricum, C. thermosaccharolyticum, Eubacterium limosum, C. ljungdahlii PETC* and *Peptostreptococcus productus.* Anaerobic bacteria known to produce hydrogen from carbon monoxide and water include *Rhodospirillum* rubrum and *Rhodopseudomonas gelatinosa.*

More specifically, bacterial species such as *Acetogenium kivui, Peptostreptococcus productus, Acetobacterium woodii*, *Clostridium thermoaceticum* and *Eubacterium limosum* produce acetate by the reaction:

4CO + 2H₂O → CH₃COOH + 2CO₂ dG = -39 kcal/reac. (1)

Many anaerobic bacteria are also known to produce acetic acid from H₂ and CO₂. These bacterial isolates include *A. kivui*, *P. productus,* and Acetobacterium *sp*., which utilize homoacetic fermentation by anaerobically oxidizing hydrogen and CO₂ according to the equation:

4H₂ + 2CO₂ → CH₃COOH + 2H₂O dG = -25 kJ/reac. (2)

*Acetobacterium woodii* and *Acetoanaerobium noterae* produce acetate from H₂ and CO₂ according to the above reaction, but in addition to acetate, A. noterae produces some propionate and butyrate. Another chemolithotrophic bacteria, *Clostridium aceticum*, produces acetate from CO₂ using a glycine decarboxylase pathway.

Some bacteria, like *A kivui*, *P. productus*, and *A woodii*, produce acetate from either CO and H₂O or H₂ and CO₂. P. productus gives particularly fast rates of conversion and demonstrates high tolerance to CO; however, this organism shows a preference to follow Equation (1) over Equation (2).

In addition to these listed bacteria, two strains of an additional clostridia which produce acetic acid or ethanol from CO and H₂O or H₂ and CO₂ have been isolated. One is a *Clostridium ljungdahlii* ERI-2 rod-shaped, gram positive, non-thermophilic anaerobe which gives superior acetic acid yields and operates at a low pH, which greatly enhances the recovery of the product. *C. ljungdahlii* ERI-2 carries out a vigorous acetogenic fermentation of glucose. It also infrequently forms spores and carries out a primarily acetogenic fermentation of hexose or H₂:CO₂. It is motile with peritrichous flagellation. This new strain of *C. ljungdahlii*, referred to as ERI-2, was isolated from a natural water source and was deposited on December 8, 1992, with the American Type Culture Collection (ATCC), Rockville, Maryland, accession no. 55380.

In preparing the products of the present invention, "mixed strains" of the bacteria enumerated hereinabove may be utilized. By mixed strains, it is meant a mixed culture of two or more anaerobic bacteria. This mixed strain, when utilized in the process described herein, produces acetic acid. It may also be utilized to produce other organic acids or salts thereof, alcohols, hydrogen, SCP, etc.

In the development of the present invention, new strains of anaerobic bacteria have been isolated which enact this conversion with high efficiency. In addition, modifications to the fermentation conditions can result in the production of ethanol instead of acetic acid in some strains. Depending on the specific microorganism(s) utilized, variables which must be considered in forming products from waste gases include nutrient constituents and concentrations, medium, pressure, temperature, gas flow rate, liquid flow rate, reaction pH, agitation rate (if utilizing a Continuously Stirred Tank Reactor), inoculum level, maximum substrate (introduced gas) concentrations to avoid inhibition, and maximum product concentrations to avoid inhibition.

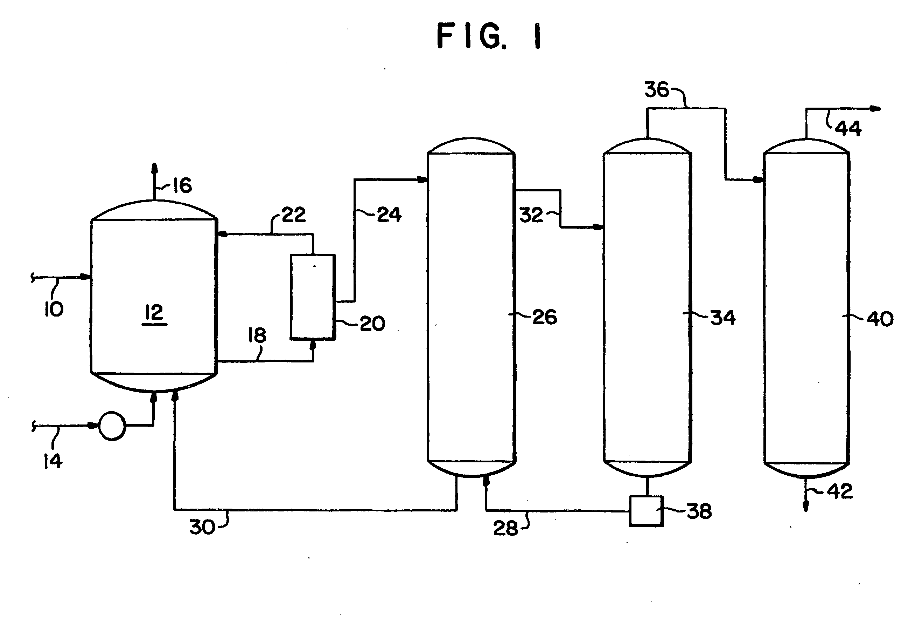

In accordance with an exemplary embodiment of the present invention and as shown in Figure 1 of the drawings, a first step in the conversion process is the preparation of nutrient media (10) for the anaerobic bacteria. The content of the nutrient media will vary based on the type of anaerobe utilized and the desired product. The nutrients are constantly fed to a bioreactor or fermenter (12), consisting of one or more vessels and/or towers of a type which includes the Continuously Stirred (CSTR), Immobilized Cell (ICR), Trickle Bed (TBR), Bubble Column, Gas Lift Fermenters, or other suitable fermentation reactor. Within the bioreactor (12) resides the culture, either single or mixed species, of anaerobic bacteria utilized in the gas conversion process. For the CSTRS, TBRs, Bubble Columns and Gas Lift Fermenters, these bacteria live dispersed throughout the liquid phase of the reactor, but for ICRs, the bacteria adhere to an internal packing medium. This packing medium must provide maximal surface area, high mass transfer rate, low pressure drop, even gas and liquid distribution, and must minimize plugging, fouling, nesting and wall channeling. Examples of such medium materials are ceramic Berl saddles, Raschig rings or other high performance packings.

The waste gases (14) are continuously introduced into the bioreactor (12). The gas is retained in the bioreactor (12) for the period of time which maximizes efficiency of the process. Exhaust gases (16), containing inert and unreacted substrate gases, are then released. The liquid effluent (18) is passed to a centrifuge, hollow fiber membrane, or other filtration device (20) to separate out microorganisms that are entrained. These microorganisms (22) are returned to the bioreactor (12) to maintain a high cell concentration which yields a faster reaction rate (cell recycle).

A next step in the process is separation of the desired biologically produced product(s) from the permeate or centrifugate (24). In the embodiment depicted in Figure 1, the permeate or centrifugate (24) is passed to an extraction chamber (26) where it is contacted with a solvent (28). The solvent (28) should have a high distribution coefficient for the desired end product, a high recovery factor, low toxicity to humans, low toxicity to the bacteria, immiscibility with water, appropriately high boiling point, and form no emulsion with the bioreactor constituents. The distribution of solute between solvent and aqueous phase will determine the thermodynamic feasibility and the amount of solvent required to remove the end product. Typical solvents include secondary and tertiary amines in a suitable solvent, tributyl phosphate, ethyl acetate, tri-octyl phosphine oxide and related compounds in a suitable co-solvent, long chain alcohols, hexane, cyclohexane, chloroform, and tetrachloroethylene.

The nutrients and materials in the aqueous phase (30) pass back to the bioreactor (12) and the solvent/acid/water solution (32) passes to a distillation column (34), where it is heated to a sufficient temperature to separate the solvent (28) from the acid and water (36). The solvent (28) passes from the distillation column (34) through a cooling chamber (38) to lower the temperature to the optimum temperature for extraction, then back-to the extraction chamber (26) for reuse. The acid and water solution (36) passes to a final distillation column (40) where the desired end product (42) is separated from the water and removed. The water (44) is recirculated for nutrient preparation.

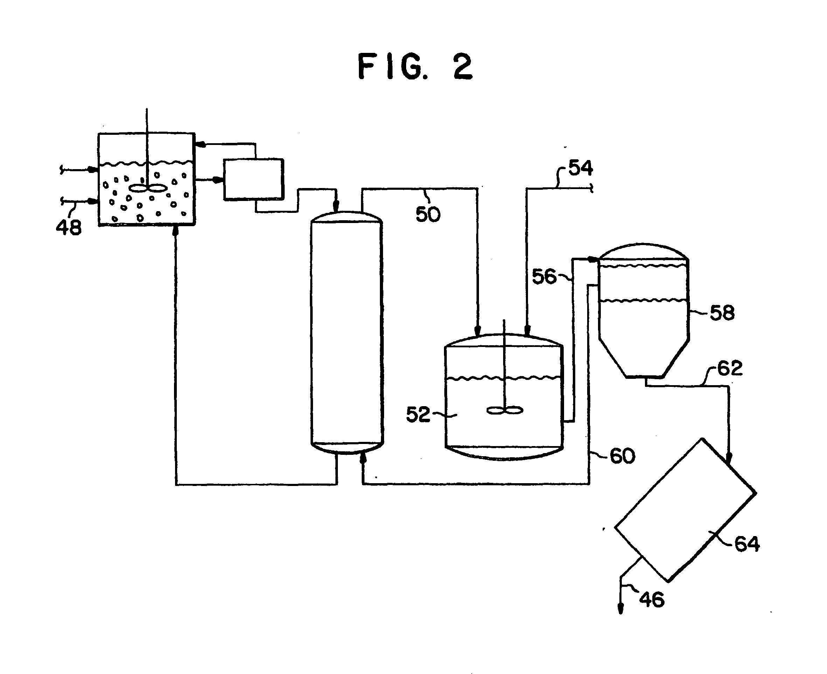

Figure 2 shows a process for the production of the road deicer calcium magnesium acetate (CMA)(46), from waste gas (48). The process is identical to the acetic acid process of Figure 1 through solvent extraction. That is, identical organisms, nutrients and process conditions are used in continuous fermentation, including the reaction vessels.
Similarly, cell recycle by hollow fiber membrane, centrifugation or other filtration device is identically employed in the process. Finally, the extraction of acetic acid in an extraction chamber, followed by recycle of the acid-free medium, is employed.

After extraction, the process for producing CMA differs greatly from the acetic acid production process of Figure 1. In the CMA process the solvent (50) containing acetic acid and a small amount of water is sent to a reaction vessel (52) for CMA production. The water content of the solvent stream is dependent upon the solvent used for acetic acid extraction. Again, solvents such as secondary and tertiary amines in a suitable co-solvent, tributyl phosphate, ethyl acetate, tri-octyl phosphine oxide and related compounds in a suitable co-solvent, long chain alcohols, hexane, cyclohexane, chloroform and tetrachloroethylene may be employed with varying success. The reaction vessel (52) for CMA is most suitably a Continuous Stirred Tank Reactor (CSTR), although other reactor systems may be employed. A mixture (54) of dolomitic lime and magnesium oxide in water is added to the solvent containing acetic acid and water. Reaction occurs to produce CMA in aqueous solution at or below the saturation level.

The CMA, water and solvent (56) are then sent to a settling device (58) to separate the aqueous and solvent phases. The solvent phase (60) is returned to the extraction chamber for recycle. The CMA/water (62) is sent to drying/pelletizing (64) to produce a pelletized CMA product.

Potassium acetate (KA) can be produced as an alternative product by substituting caustic potash (or potassium oxide) for the dolomitic lime. Since KA is produced as a 50 percent aqueous solution, drying and pelletizing are not required.

Thus in accordance with the present invention it is now possible to produce valuable acetic acid and acetic acid salts by a gaseous substrate fermentation, not only reducing consumption of valuable chemical feedstocks, but also removing hazardous atmospheric pollutants from the waste gas streams of many industries. Previous processes to derive these chemicals biologically were based on fermentation of sugars.

In the processes described hereinabove, it is preferred that the process is conducted at higher than 1 atmosphere. Preferably, it is preferred that it be conducted at pressures up to 30 atmospheres and more preferably up to 20 atmospheres and most preferably up to 15 atmospheres.

The following specific examples are submitted to illustrate but not to limit the present invention. Unless otherwise indicated, all parts and percentages in the specification and claims are based upon volume.

### EXAMPLE 1

### PRODUCTION OF ACETIC ACID FROM CARBON BLACK WASTE GASES

This example is directed to a process utilized to convert waste gas of a composition which matches that of the furnace exhaust of carbon black manufacture to acetic acid. The waste gas has a composition of about 13 percent carbon monoxide, 14 percent hydrogen, and 5 percent carbon dioxide, with the remaining 68 percent largely nitrogen with traces of oxygen and sulfur compounds. The waste gases are produced as the result of partial oxidation of gas or oil with insufficient air to form amorphous carbon, with about 1.2 pounds of carbon monoxide produced per pound of elemental carbon. These waste gases form a serious atmospheric contamination problem and also represent a valuable chemical feedstock resource not presently being recovered.

In the development of the present process, two distinct routes to produce acetic acid from carbon black waste gases were studied. The direct route converts CO and H₂O or H₂ and CO₂ directly into acetic acid according to Equations (1) and (2), respectively. An indirect route involves the conversion of CO and H₂O into H₂ and CO₂ by the water gas shift reaction, followed by production of acetic acid from H₂ and CO₂. This indirect route was found to be a less efficient utilization of the technology.

The acetogens tested are summarized in Table 1. Among these bacteria that produce acetic acid directly from CO, *A. kivui* and the newly isolated strain, *C. ljungdahlii* ERI-2, show far superior rates for both CO and H₂ utilization. Further experimentation proceeded using these two anaerobic bacteria.

There are obvious advantages to the bacteria utilizing carbon monoxide and hydrogen simultaneously. This would afford the most efficient use of the waste gases and remove the greatest amount of atmospheric pollutants.

### Bench Scale Operation of the Described Process to Produce Acetic Acid

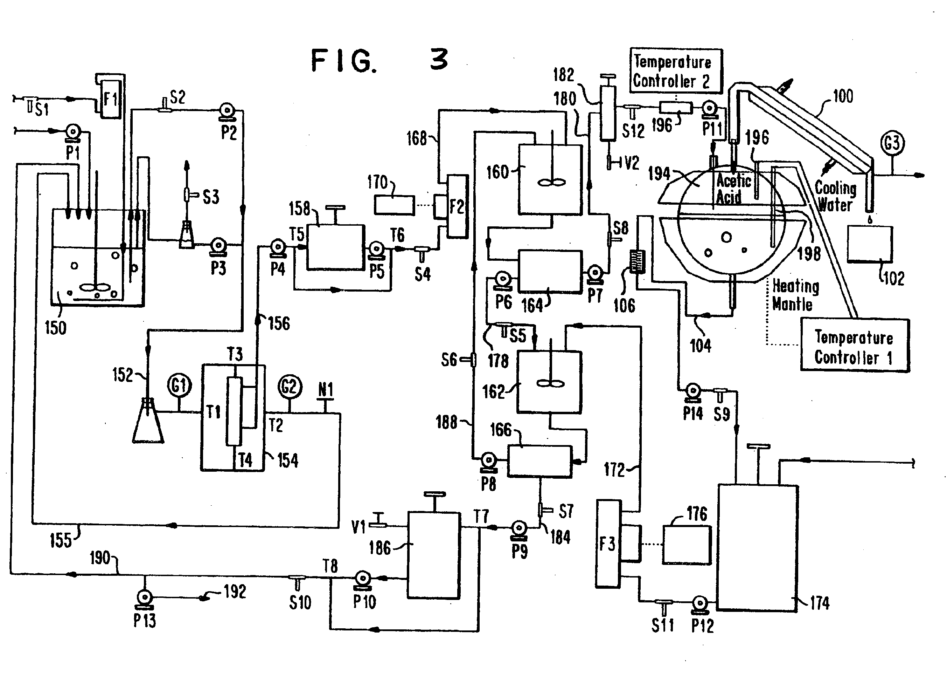

As shown in Figure 3 of the drawings and in accordance with one embodiment of the present invention, a bench scale continuous conversion system is shown to include a BioFlo IIC fermentor (150) from New Brunswick Scientific Co., Inc., Edison, NJ. The fermentor (150) is equipped with an agitation motor, pH controller, foam controller, thermostat, dissolved oxygen probe, nutrient pump, and 2.5 L culture vessel. The working volume is variable (1.5-2.0 L). Other variable operational parameters include medium feeding rate (Dilution rate), gas flow rate (Gas retention time), agitation (rpm). The vented or exhaust gases exit the fermentor (150) through a condenser fixed to a vented hood via a water trap and a sampling port.

The culture broth (152) is recycled through a cross-flow hollow fiber module (154) by a peristaltic pump (from Cole Parmer). The recycling rate is about 80-100 mL/min. The hollow fiber module (154) has the following characteristics; the surface area is 0.35 ft² (325 cm²), the pore size is 0.2 µm and the lumen diameter is 1 mm. The permeate (156) is pumped to a storage tank (158) (Feed storage). The culture cells are returned to the fermenter along line (155).

A counter-current acetic acid extraction system, including two stage mixer and settler components includes first and second mixers (160) and (162) and first and second settling tanks (164) and (166). The permeate (168) from storage (158) is pumped to mixer (160) through a flow controller (170). The solvent (172) is pumped to mixer (162) from solvent storage (174) through a flow controller (176). Both mixer (160) and mixer (162) are equipped with a stirring mechanism to achieve good mixing of aqueous phase and solvent phase. The mixture of both phases from the mixers (160) and (162) is led to settlers (164) and (166), respectively. The phase separation is accomplished in the settlers. The aqueous phase (178) from settler (164) is pumped to mixer (162), the solvent phase (180) from settler (164) is pumped to a separator (182), the aqueous phase (184) from settler (166) is pumped to raffinate storage (186), and the solvent phase (188) from settler (166) is pumped to mixer (160). The raffinate is recycled to the CSTR (150) along a line (190). This recycle line (190) is partially bled at (192) to remove inhibiting factors.

The solvent (180) loaded with acetic acid is pumped to a distillation flask (194) through a preheater (196). The distillation flask (194) is equipped with two thermocouples (196) and (198) to monitor and control temperature in the liquid phase and gas phase. The heating temperature for distillation is set to achieve maximum vaporization of the acetic acid. The acetic acid vapors are condensed in a condenser (100) and collected in a flask (102). The stripped solvent (104) is pumped through a cooling coil (106) to solvent storage (174).

A bench scale operation of the described process as diagrammed in Figure 3 was fabricated in the laboratory to determine quantitative yields under optimized conditions. The nutrient mixture fed to the culture was as follows:
1. 80.0 ml of a salt, composed of

| | |
|---|---|
| KH₂PO₄ | 3.00 g/L |
| K₂HPO₄ | 3.00 g/L |
| (NH₄)₂SO₄ | 6.00 g/L |
| NaCl | 6.00 g/L |
| MgSO₄. 2H₂O | 1.25 g/L |

2. 1.0 g of yeast extract
3. 1.0 g of trypticase
4. 3.0 ml of PFN (Pfenning) trace metal solution

| | |
|---|---|
| FeCl₂ * 4H₂O | 1500 mg |
| ZNSO₄ * 7H₂O | 100 mg |
| MnCl₂ * 4H₂O | 30 mg |
| H₃BO₃ | 300 mg |
| CoCl₂ * 6H₂O | 200 mg |
| CuCl₂ * H₂O | 10 mg |
| NiCl₂ * 6H₂O | 20mg |
| NaMoO₄ * 2H₂O | 30mg |
| Na₂SeO₃ | 10 mg |
| Distilled water | 1000 ml |

5. 10.0 ml of B vitamins

| | |
|---|---|
| Pyridoxal HCl | 10 mg |
| Riboflavin | 50 mg |
| Thiamine HCl | 50 mg |
| Nicotinic acid | 50 mg |
| Ca-D-Pantothenate | 50 mg |
| Lipoic Acid | 60 mg |
| P-aminobenzoic acid | 50 mg |
| Folic acid | 20 mg |
| Biotin | 20 mg |
| Cyanocobalamin | 50 mg |
| Distilled water | 1000 ml |

6. 0.5 g of Cysteine HCl
7. 0.06 g of CaCl₂.2H₂O
8. 2.0 g of NaHCO₃
9. 1.0 ml of Resazurin (0.01%)
10. 920.0 ml of distilled water For use with *A kivui*, the nutrient solution was pH adjusted to 6.6, whereas for the new strain, *C. ljungdahlii* ERI-2, the pH was adjusted to 4.9. The ability to operate at a lower pH is a great advantage in acetic acid recovery. The solution was then sparged for 20 minutes with a 20% CO₂ and 80% N₂ atmosphere, then transferred anaerobically and autoclaved for 15 minutes.

Numerous experiments were carried out with both Continuous Stirred Reactors (CSTR) and Immobilized Cell Reactors (ICR). The results obtained are exemplified in the following data.

### CSTR Experiments Utilizing the bacterial Strains A. kivui and C. ljungdahlii ERI-2

The bench scale system operating with the CSTR and the anaerobic bacteria, *C. ljungdahlii* ERI-2 and *A. kivui*, consisted of a New Brunswick Scientific Bioflo IIc fermenter, a hollow fiber membrane unit for cell recycle, and extraction and distillation columns. Nutrient mixture was fed into the bioreactor at a rate of 3.2 cubic centimeters per minute. Capacity of the reactor was 2.5 liter, within which a constant fluid level of 1.5 liters was maintained. The fluid was agitated at variable rates of up to 1000 revolutions per minute with gas introduced at a rate of approximately 500 cubic centimeters per minute. Optimal gas retention times were in the range of three minutes. The gas feed varied with its uptake by the bacteria, which was in turn a function of the cell density.

The liquid from the bioreactor was passed to the hollow fiber membrane at a rate of 55 to 70 milliliters per minute. From the hollow fiber membrane, permeate was gathered at a rate of 1.5 milliliters per minute. Analysis of this permeate indicates the acetic acid/acetate concentration at this stage to range in excess of 20 grams per liter. Operating at a pH of 4.9, 42 percent of this product was in the acid form using *C*. *ljungrdahlii* ERI-2. For *A*. *kivui*, the acid yield was only 1.4 percent. Results of various runs for the two bacteria, including conversion rates and product yields are summarized in Tables 2 and 3.

### ICR Experiments Utilizing the Bacterial Strain C. ljungdahlii ERI-2.

An Immobilized Cell Reactor (ICR), consisting of a 2 inch (5.08 cm) outside diameter by 24 inch (61 cm) tall glass tube packed with fabric to support the cells and Enkamat 7020 as an immobilizing medium was also tested in the acetic acid production process. With C. ljungdahlii ERI-2 as the acetogenic anaerobe, 100 percent of the carbon monoxide and 79 percent of the hydrogen were converted at a gas retention time of 20 minutes. Acetic acid concentrations in the removed liquid were approximately 6.0 grams per liter. Results of the ICR studies are summarized in Table 4.

The ICR has a certain attractiveness on an industrial scale in that the energy costs to operate the reactor are reduced significantly. The proper selection of packing materials, solution phases, and pressures may yield production approaching that of the CSTR.

### Acetic Acid Recovery

Various solvents were tested for recovering acetic acid from the permeate, the results are summarized in Table 5. Tributyl phosphate was identified as having both a high distribution coefficient and a high boiling point. The solvent and permeate from the cell separator were commingled in a two stage extraction process. Alternatively, an extraction column could be used. Permeate was introduced into a 3 liter flask where it was mixed with incoming solvent. A ratio of 1 part solvent to 1 part permeate worked well and gave high recovery rates. The combined fluids were passed from the mixer to a 4 liter settling chamber where the solvent/acetic acid mixture separate as a lower density phase from the water and nutrients. Retention times of approximately 15 minutes were used in the settling tanks. The lower density phase was extracted and fed to a distillation flask. The raffinate was passed from the first settler to a second mixer where it was contacted again with solvent, then removed to a second settling chamber. This allowed for more complete extraction of the acetic acid; acid recovery increased from 82 percent to greater than 96 percent using tributyl phosphate. The solvent/acetic acid mixture from this settler was returned to the first mixer, while the raffinate of water and organics was passed back to the bioreactor.

The distillation unit was a 5 liter flask with a boiling mantle. A common distillation column, with reflux, could be used for complete acid recovery. Because of the high boiling point of tributyl phosphate, nearly complete recovery is accomplished in one step. The solvent/acetic acid mixture was heated to 120 degrees C, with the acetic acid collected overhead in a condensing coil. In this single stage system, distillation efficiencies of 70 percent were achieved.

Solvent mixtures were also tried and distribution coefficients of mixed solvents are summarized in Table 6.

### EXAMPLE 2

### PRODUCTION OF ACETIC ACID FROM CARBON BLACK WASTE GASES

### AT HIGHER PRESSURES

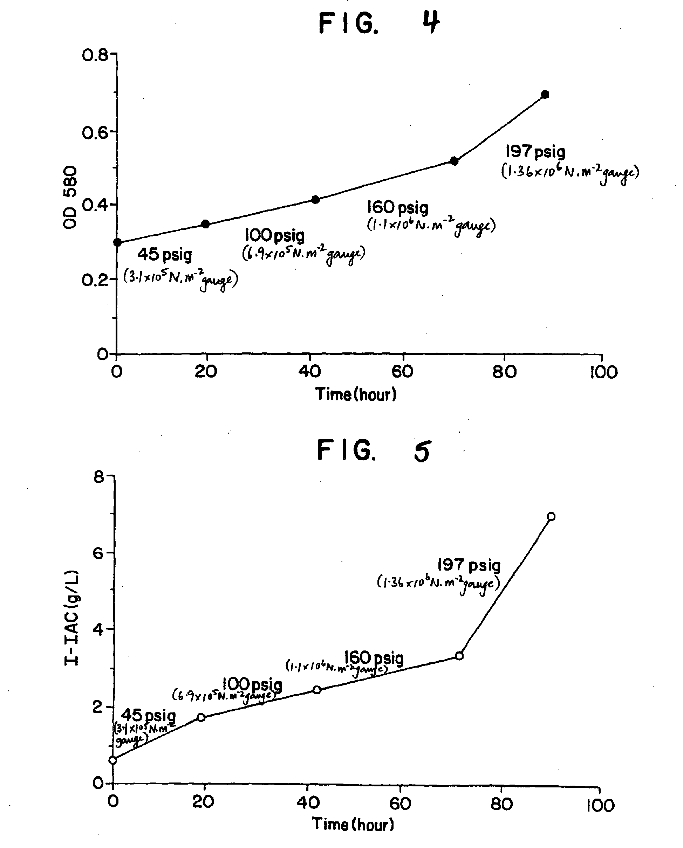

Mass transport in the cellular reactions can be further enhanced by operating the system at increased pressures. Simple batch experiments were carried out to test the dynamics of this system. It was found that reaction rates increased in linear proportion to the pressure, with a corresponding reduction in effective retention time. Another advantage to operating at increased pressure is that reactor volume can also be reduced in linear fashion, i.e. operation at 10 atmospheres (1 x 10⁶ N.m⁻²) pressure requires a reactor with one tenth the volume of a reactor operating at 1 atmosphere (1 x 10⁵ N.m⁻²). Figures 4 and 5 show the increase in cell density and acetic acid concentration, respectively, with the increased pressure. This acetic acid concentration far exceeds typical batch concentrations for a batch reactor at atmospheric pressure.

### EXAMPLE 3

### PRODUCTION OF ACETIC ACID FROM CARBON BLACK WASTE GASES WITH SURFACTANTS

Mass transport is also increased by the use of surfactants. Table 7 presents the results of carbon monoxide uptake tests performed on C. ljungdahlii ERI-2 in the pressure of various commercial surfactants. In each case, the control value of 100 (percent) represents CO uptake in batch fermentation, and the sample value, the percentage of the control in batch fermentation in the presence of the surfactant.

**TABLE 1**

| ACETOGENIC BACTERIA TESTED FOR CO, H₂, AND CO₂ CONVERSION | |
|---|---|
| Bacterial Route | Simultaneous Consumption of CO and H₂ |
| Direct Route | |
| P. productus | No |
| E. limosum | No |
| A. noterae | No |
| C. aceticum | No |
| C. thermoaceticum | No |
| S. sphaeroides | No |
| A. woodii | Yes |
| A. kivui | Yes |
| C. ljungdahlii ERI-2 | Yes |

| Indirect Route | |
|---|---|
| R. gelatinosa | No |
| R. rubrum | No |

**Table 2. Summary of ERI2 Experiments in the CSTR with Cell Recycle**

| Gas Reten. Time | Liquid Dilution Rate | Agitat. Rate | Percent Gas Conversion | | Dry Cell Weigh Conc. | Product Concentration | | Specific Productivities | |
|---|---|---|---|---|---|---|---|---|---|
| | | | | | | HAC | ETOH | | |
| (min) | (hr⁻¹) | (rom) | CO | H₂ | (g/L) | (g/L) | (g/L) | (g/L hr) | (g/g hr) |
| 9.30 | 0.056 | 750 | 80.75 | 74.5 | 2.3 | 9.7 | 0.07 | 0.43 | 0.18 |
| 9.28 | 0.055 | 750 | 82.1 | 72.0 | 3.32 | 9.56 | 0.094 | 0.52 | 0.16 |
| 6.14 | 0.061 | 750 | 73.6 | 46.5 | 4.11 | 12.78 | 0.125 | 0.78 | 0.19 |
| 6.4 | 0.08 | 750 | 74.8 | 49.6 | 5.02 | 12.98 | 0.125 | 1.05 | 0.19 |
| 4.74 | 0.087 | 750 | 68.5 | 37.2 | 4.79 | 12.38 | 0.125 | 1.08 | 0.23 |
| 4.91 | 0.10 | 750 | 68.8 | 50.2 | 4.53 | 10.73 | 0.05 | 1.08 | 0.24 |
| 4.05 | 0.102 | 750 | 65.5 | 58.1 | 5.27 | 11.49 | 0.076 | 1.17 | 0.22 |
| 3.98 | 0.103 | 900 | 74.3 | 67.9 | 6.17 | 12.73 | 0.1 | 1.31 | 0.21 |
| 2.89 | 0.117 | 900 | 66.1 | 33.9 | 5.91 | 11.69 | 0.04 | 1.38 | 0.23 |
| 3.28 | 0.105 | 1000 | 74.6 | 51.3 | 7.30 | 12.83 | 0.13 | 1.35 | 0.18 |
| 3.22 | 0.125 | 1000 | 73.1 | 54.0 | 10.25 | 13.57 | 0.08 | 1.71 | 0.17 |
| 2.65 | 0.13 | 1000 | 63.9 | 44.0 | 11.0 | 14.63 | 0.12 | 1.90 | 0.17 |
| 2.3 | 0.134 | 1000 | 66.0 | 38.7 | 11.1 | 20.59 | 0.113 | 2.77 | 0.25 |
| 2.7 | 0.11 | 1000 | 72.7 | 67.7 | 8.37 | 25.62 | 0.27 | 2.88 | 0.34 |
| 2.4 | 0.11 | 1000 | 68.6 | 63.3 | 9.88 | 25.62 | 0.36 | 2.95 | 0.30 |
| 2.55 | 0.122 | 1000 | 72.1 | 67.4 | 9.82 | 25.62 | 0.72 | 3.12 | 0.32 |
| 3.0 | 0.13 | 1000 | 76.6 | 73.3 | 12.4 | 22.33 | 0.52 | 2.90 | 0.23 |

**Table 3. Summary of A. kivui Experiments in the CSTR with Cell Recycle**

| Gas Reten. Time | Liquid Dilution Rate | Agitat. Rate | Percent Gas Conversion | | Dry Cell Weigh Conc. | Product Concentration | Specific Productivities | |
|---|---|---|---|---|---|---|---|---|
| (min) | (hr⁻¹) | (rom) | CO | H₂ | (g/L) | (g/L) | (g/L hr) | (g/g hr) |
| 5.0 | 0.058 | 750 | 67.8 | 44.2 | 4.00 | 16.15 | 0.96 | 0.24 |
| 4.4 | 0.958 | 750 | 65.7 | 38.5 | 4.8 | 16.63 | 0.94 | 0.19 |
| 4.3 | 0.058 | 900 | 71.3 | 40.7 | 4.5 | 17.03 | 0.99 | 0.21 |
| 3.72 | 0.058 | 900 | 69.0 | 37.3 | 5.14 | 19.16 | 1.13 | 0.22 |
| 3.72 | 0.076 | 900 | 70.3 | 41.1 | 5.28 | 16.17 | 1.21 | 0.23 |
| 3.2 | 0.076 | 900 | 65.4 | 41.4 | 5.71 | 16.85 | 1.23 | 0.23 |
| 2.8 | 0.076 | 900 | 61.5 | 29.1 | 5.00 | 16.16 | 1.22 | 0.23 |
| 2.8 | 0.076 | 1000 | 69.5 | 36.3 | 5.8 | 18.58 | 1.62 | 0.29 |
| 2.8 | 0.11 | 1000 | 70.2 | 41.6 | 5.9 | 18.4 | 1.84 | 0.36 |
| 2.2 | 0.11 | 1000 | 64.0 | 28.0 | 7.2 | 16.5 | 2.1 | 0.3 |

**Table 4. Fabric ICR Performance with ERI2**

| Liquid Dilution Rate | Gas Retention Time | H₂ Conversion | CO Conversion | Cell Concen. | Product Concentration | |
|---|---|---|---|---|---|---|
| | | | | | HAC | ETCH |
| (hr) | (min) | (g) | (g) | (g/L) | (g/L) | (g/L) |
| 0.23 | 4.83 | 38.62 | 54.66 | .125 | 3.221 | .778 |
| | 7.41 | 49.15 | 70.87 | .120 | 2.690 | .620 |
| | 11.66 | 51.31 | 80.61 | .067 | | |
| | 13.61 | 56.67 | 83.93 | .064 | 2.099 | .201 |
| | | | | | | |
| 0.17 | 6.39 | 48.15 | 73.27 | .161 | 3.382 | 1.365 |
| | 11.21 | 68.96 | 92.82 | .143 | 3.189 | 495 |
| | 55.44 | 83.13 | 98.27 | .112 | .813 | .058 |
| | | | | | | |
| 0.12 | 6.26 | 43.89 | 70.76 | .094 | 3.864 | 1.689 |
| | | | | | | |
| 0.09 | 7.87 | 42.40 | 79.72 | .095 | 4.423 | 2.733 |
| | 19.82 | 59.63 | 92.92 | .102 | | |
| | | | | | | |
| 0.03 | 22.14 | 55.01 | 94.21 | .071 | 4.878 | 2.631 |
| | 29.00 | 78.60 | 100 | .018 | 5.604 | 2.743 |
| | 60.48 | 83.33 | 100 | | | |

**Table 5. Acetic Acid Distribution Coefficient Study**

| Solvent | Equilibrium Aqueous Acetic Acid Concentration. g/L | Acetic Acid Distribution Coefficients |
|---|---|---|
| Hexane | 6.559 | 0.0 |
| Decane | 5.968 | 0.08 |
| Chloroform | 5.128 | 0.09 |
| Kerosene | 4.648 | 0.11 |
| Hexadecane | 5.866 | 0.13 |
| Dodecane | 4.654 | 0.13 |
| Dodecyl acetate | 5.787 | 0.15 |
| Dibutyl phosphate | 4.615 | 0.18 |
| Oleyl alcohol | 5.114 | 0.28 |
| Trioctylamine | 3.785 | 0.31 |
| Undecyl alcohol | 4.528 | 0.40 |
| Ethyl acetate | 4.550 | 0.41 |
| Ethyl butyrate | 4.665 | 0.42 |
| Dexyl alcohol | 3.890 | 0.42 |
| Octanol | 4.358 | 0.45 |
| Nonyl alcohol | 3.470 | 0.55 |
| 2-ethyl-1-hexanol | 3.308 | 0.77 |
| 3-methyleyclonexanol | 2.110 | 1.26 |
| Cyclohexanone | 2.702 | 1.66 |
| Tributyl Phosphate | 1.657 | 2.38 |

**Table 6. Distribution Coefficients of Mixed Solvents**

| solvent Mix | Distribution Coefficients | Percent Increase |
|---|---|---|
| Oleyl Alcohol (10cc) | 0.17 | |
| Oleyl Alcohol (10cc) + Cyc (1cc) | 0.31 | 72 |
| Oleyl Alcohol (10cc) + TBP (1cc) | 0.29 | 61 |
| | | |
| Alcohol (10cc) + Cyc (2cc) | 0.45 | 150 |
| Oleyl Oleyl Alcohol (10cc) + TBP (2cc) | 0.42 | 133 |
| | | |
| Oleyl Alcohol (10cc) + Cyc (3cc) | 0.36 | 100 |
| Oleyl Alcohol (10cc) + TBP (3cc) | 0.42 | 133 |
| | | |
| Oleyl Alcohol (10cc) + Cyc (4cc) | 0.35 | 94 |
| Oleyl Alcohol (10cc) + TBP (4cc) | 0.40 | 122 |
| | | |
| Oleyl Alcohol (10cc) + Cyc (6cc) | 0.52 | 188 |
| Oleyl Alcohol (10cc) + TBP (6cc) | 0.65 | 261 |
| | | |
| Oleyl Alcohol (10cc) + Cyc (7cc) | 0.69 | 283 |
| Oleyl Alcohol (10cc) + TBP (7cc) | 0.74 | 311 |

**TABLE 7. CO CONSUMPTION BY ERI-2 IN THE PRESENCE OF SURFACTANTS**

| | | Control* | With Surfactant |
|---|---|---|---|
| DNAP (0.1%, v/v | | 100 | 0 |
| Nondiet P-40 (0.1%, v/v) | | 100 | 0 |
| Tergitol NP-10 (0.1%, v/v) | | 100 | 0 |
| Tergitol Min Foam 1X (0.1%, v/v) | | 100 | 0 |
| Tergitol TMN-10 (0.1%, v/v) | | 100 | 0 |
| Triton X-15 (0.1%, v/v) | | 100 | 0 |
| Triton X-100 (0.1%, v/v) | | 100 | 0 |
| Triton X-114 (0.1%, v/v) | | 100 | 0 |
| Triton N-101 (0.1%, v/v) | | 100 | 5.83 |
| Triton X-405 (0.1%, v/v) | | 100 | 7.82 |
| Tergitol 8 (0.1%, v/v) | | 100 | 12.15 |
| Triton N-42 (0.1%, v/v) | | 100 | 42.90 |
| Witconol NS-500K (0.01%, w/v) | | 100 | 79.08 |
| Tween 85 (0.1%, v/v) | | 100 | 82.16 |
| Witconol H-33 (0.1%, v/v) | | 100 | 90.12 |
| Witconol 6903 (0.1%, v/v) | | 100 | 92.39 |
| Tween 80 (0.1%, v/v) | | 100 | 97.15 |
| Arlacel 83 (0.1%, v/v) | | 100 | 97.43 |
| Span 80 (0.1%, v/v) | | 100 | 99.12 |
| Tyloxapol (0.1%, v/v) | | 100 | 104.86 |
| Witconol 5906 (0.1%, v/v) | | 100 | 108.42 |
| Span 85 (0.1%, v/v) | | 100 | 124.85 |
| W-1 (0.001%, w/v) | First time | 100 | 105.89 |
| | Second time regas | 100 | 0 |
| Brij 96 (0.004%, w/v) | First time | 100 | 107.98 |
| | Second time regas | 100 | 0 |

### EXAMPLE 4

### PRODUCTION OF CMA FROM CARBON BLACK WASTE GAS

Carbon black waste gas containing about 14 percent CO, 17 percent H₂ and 4 percent CO₂, as the major components in N₂ is sparged into a 160L continuous stirred tank reactor, maintained at 6 atm (6 x 10⁵ N.m⁻²) 37°C, and containing *Clostiridium ljungdahlii* isolate ERI-2 ATCC deposit 55380. The waste gases are produced as the result of partial oxidation of hydrocarbons with insufficient air to form amorphous carbon, with about 1.2 Kg of carbon monoxide produced per Kg of elemental carbon. These waste gases form a serious atmospheric contamination problem and also represent a valuable chemical feedstock resource not presently being recovered. The gas retention time (defined as the ratio of the reactor volume to the gas flow rate at standard conditions) is maintained at 0.52 min. An aqueous liquid medium containing water, base salts, B-vitamins, a nitrogen source and a sulfide source is fed to the reactor at a liquid dilution rate (defined as the ratio of the liquid flow rate to the reactor volume) of 1.05 hr⁻¹. The agitation rate in this reactor is 322 rpm, the temperature is 37°C and the operating pH is 5.03. Under these conditions, the conversion of CO was 83 percent and the conversion of H₂ was 54 percent. A hollow fiber membrane cell recycle unit is used to maintain a cell concentration of 10.5 g/L inside the reactor. The dilute acetic acid/acetate product stream from the reactor containing 13.2 g/L acetic acid/acetate is sent to a three stage countercurrent extraction device, where it is extracted with solvent. The solvent to feed ratio is 1 to 4. The acetic acid in the acetic acid/acetate product stream is 3.7 g/L. The acetic acid concentration in the solvent leaving the extractor is 16.7 g/L. Water (medium) from extraction is sent back to the fermenter as recycle.

Dolomitic lime/MgO is added to the acetic acid directly in the solvent phase to form CMA. After reaction the saturated CMA solution is sent to drying and pelletizing 1.15 Kg of CMA containing a Ca²⁺/Mg²⁺ molar ratio of 3/7 is formed per Kg of acetic acid.

### EXAMPLE 5

### PRODUCTION OF ACETIC ACID FROM CARBON BLACK WASTE GAS

Carbon black waste gas containing about 14 percent CO, 17 percent H₂ and 4 percent CO₂ in N₂ is sparged into a 144 L trickle bed reactor operating at 1.58 atm (1.6 x 10⁵ N.m⁻²), 37°C and containing *Clostridium Ijungdahlii* isolate ERI-2 ATCC deposit 55380. A trickle bed reactor is a column packed with a commercial packing such as Raschig rings or Berl saddles in which liquid and gas are contacted with each other due to flow through the column. In the present example, the liquid and gas both enter the column from the top in a cocurrent fashion, although countercurrent flow (gas entering the bottom, liquid entering the top) is possible. The gas retention time is maintained at 0.46 min and the liquid medium dilution rate is 0.57 hr⁻¹. The liquid medium contains the same constituents as in Example 1. Agitation in the reactor is provided by liquid recirculation, using a recirculation rate of 60 gpm. The operating pH in the reactor is 5.05. Under these conditions, the CO conversion is 57 percent and the H₂ conversion is 58 percent. A hollow fiber unit is used to maintain a cell concentration of 13.6 g/L inside the reactor.

The dilute acetic acid/acetate product stream containing 6.4 g/L combined acetic acid/acetate and 2 g/L acetic acid is sent to a three stage countercurrent extraction column. The solvent to feed ratio is 1:4. The acetic acid in the solvent leaving the extractor is 10 g/L. Water (medium) from the extraction unit is sent back as recycle to the reactor.

The solvent containing the acetic acid is sent to distillation to recover the acid and solvent. A vacuum solvent distillation column and an acetic acid distillation column are used in the separation. Glacial acetic acid is produced as the final product.

### EXAMPLE 6

### PRODUCTION OF POTASSIUM ACETATE FROM CARBON BLACK WASTE GAS

The carbon black waste gas of Example 4 is used to make potassium acetate instead of CMA. All fermentation and solvent extraction conditions remain the same. Caustic potash (potassium oxide) is used to react with the acetic acid to form a 50 percent solution of potassium acetate directly in the solvent phase.

### EXAMPLE 7

### PRODUCTION OF SCP FROM COKE OVEN WASTE GAS

A coke oven waste gas containing about 6 percent CO, 2 percent CO₂, 57 percent H₂, 5 percent N₂, and 27 percent gaseous hydrocarbon is fed to a continuous stirred tank reactor with cell recycle as described previously in Example 4. The reactor is used to produce a product such as dilute acetic acid or ethanol. In addition, the cell concentration inside the reactor is 13.6 g/L. These cells (microorganisms) can be harvested to produce bacterial single cell protein as an animal feed. A purge stream from the reactor containing cells is sent to a dryer to process dry single cell protein.

### EXAMPLE 8

### PRODUCTION OF H₂ FROM REFINERY WASTE GAS

Refinery waste gas containing about 45 percent CO, 50 percent H₂ and 5 percent CH₄ is sparged into a 1L CSTR operating at 50°C and a few inches of water pressure containing *Bacillus smithii* isolate ERIH2 which was deposited on March 18, 1993 with the American Type Culture Collection, Rockville, Maryland and given deposit accession no. 55404. The medium to the reactor is 1.0 g/L corn steep liquor. CO in the waste gas is converted along with water to CO₂ and H₂. With a 90 percent conversion, the exit gas stream contains 3.2 percent CO, 64.4 percent H₂, 28.8 percent CO₂ and 3.6 percent CH₄. The CO, CO₂ and CH₄ are removed from the gas stream by solvent extraction.

Thus, it will be appreciated that as a result of the present invention, a highly effective improved process for converting waste gases to acetic acid or its salts is provided by which the principal objective, among others, is completely fulfilled. It is contemplated and will be apparent to those skilled in the art from the preceding description and accompanying drawings that modifications and/or changes may be made in the illustrated embodiments without departure from the present invention. Accordingly, it is expressly intended that the foregoing description and accompanying drawings are illustrative of preferred embodiments only, and are not limiting, and that the scope of the present invention be determined by reference to the appended claims.

## Claims

1. A process for producing acetic acid, comprising:
(a) providing a continuous flow of gas comprising:
(i) a gas comprising carbon monoxide;
(ii) a gas comprising carbon monoxide and hydrogen; or
(iii) a gas comprising hydrogen and carbon dioxide;
into a bioreactor, said bioreactor containing an aqueous nutrient medium and the anaerobic bacterium *C*. *ljungdahlii* ERI-2, ATCC No. 55380;
(b) directing a flow of said aqueous nutrient medium into said bioreactor; and
(c) fermenting said gas and said nutrient medium using said bacterium at a low pH under conditions that permit converting the gas to said acetic acid in a broth.

2. The process according to claim 1, further comprising releasing exhaust gases from said bioreactor.

3. The process according to claim 1, further comprising separating said bacterium from said broth.

4. The process according to claim 3, further comprising returning said removed bacterium to said bioreactor.

5. The process according to claim 1, wherein a solvent phase containing acetic acid and water and an aqueous phase containing nutrients are formed in an extraction chamber.

6. The process according to claim 5, further comprising separating from said solvent phase containing solvent, acetic acid and water, an aqueous phase containing nutrients, and passing said aqueous phase containing nutrients and materials to said bioreactor.

7. The process according to claim 6, further comprising distilling said solvent phase to separate acetic acid and water from said water-immiscible solvent.

8. The process according to claim 7, further comprising returning said water-immiscible solvent to said extraction chamber.

9. The process according to claim 8, further comprising separating said acetic acid from said water in a final distillation column and recycling said water to said bioreactor.

10. The process according to any of claims 1 to 9, wherein the gas (i) or (ii) further contains carbon dioxide.

11. The process according to any of claims 1 to 10, wherein said gas further comprises one or more of nitrogen and methane.

12. The process according to any of claims 1 to 11, wherein said gas is produced by an industrial process selected from the group consisting of the manufacture of carbon black, ammonia, methanol or coke or petroleum refining.

13. The process according to any of claims 1 to 12, wherein said bioreactor is a continuously stirred tank reactor, an immobilized microbial cell bioreactor, a trickle bed bioreactor, a bubble column bioreactor, or a gas lift bioreactor.

14. The process according to any of claims 1 to 13, wherein said bioreactor is maintained at a pressure of greater than one atmosphere (1 x 10⁵ N.m⁻²).

15. The process according to any of claims 1 to 13, wherein said process is performed at up to 15 atmospheres (1.5 x 10⁶ N.m⁻²) of pressure.

16. The process according to claim 3, wherein said separating is accomplished by centrifugation, hollow fiber membrane filtration, settling or ultrafiltration.

17. The process according to any of claims 1 to 16, wherein said bioreactor further comprises another anaerobic acetogenic bacterium selected from the group consisting of *Acetobacterium kivui, A. woodii, Butyribacterium methylotrophicum, Clostridium aceticum, C. acetobutylicum, C. formoaceticum, C. kluyveri, C. ljungdahlii PETC, C. thermoaceticum, C. thermocellum, C. thermohydrosulfuricum, C. thermosaccharolyticum, Eubacterium limosum,* and *Peptostreptococcus productus.*

18. The process according to claim 1, further comprising contacting the acetic acid with dolomitic lime and magnesium oxide and drying, thereby producing calcium magnesium acetate.

19. The process according to claim 1, further comprising contacting the acetic acid with caustic potash, thereby producing potassium acetate.

20. The process according to any of claims 1 to 19, wherein the pH in the bioreactor is 4.9.

21. The process according to claim 1, further comprising:
(d) removing continuously a portion of said broth containing acetic acid from said bioreactor;
(e) contacting said removed broth containing the acetic acid with a water-immiscible solvent having an affinity for the acetic acid; and
(f) optionally distilling the acetic acid from said solvent.

22. The process according to claim 21, wherein after step (e) or (f), the acetic acid is contacted with dolomitic lime and magnesium oxide and dried, thereby producing calcium magnesium acetate.

23. The process according to claim 21, wherein after step (e) or (f), the acetic acid is contacted with caustic potash, thereby producing potassium acetate.

24. The process according to any of claims 1 to 23, wherein said bioreactor further contains a surfactant which increases the consumption of carbon monoxide by said bacterium.

25. A biologically pure culture of the microorganism *Clostridium ljungdahlii* ERI-2 ATCC No. 55380.

## Patentansprüche

1. Verfahren zum Herstellen von Essigsäure, umfassend:
(a) Bereitstellen eines kontinuierlichen Stroms von Gas, umfassend:
(i) ein Kohlenmonoxid aufweisendes Gas;
(ii) ein Kohlenmonoxid und Wasserstoff aufweisendes Gas; oder
(iii) ein Wasserstoff und Kohlendioxid aufweisendes Gas;
in einem Bioreaktor, wobei der Bioreaktor ein wässriges Nährstoffmedium und das anaerobe Bakterium *C*. *ljungdahlii* ERI-2, ATCC Nr. 55380 enthält;
(b) Leiten eines Stroms des wässrigen Nährstoffmedium in den Bioreaktor; und
(c) Fermentieren des Gases und des Nährstoffmediums unter Verwendung des Bakteriums bei einem niedrigen pH-Wert unter Bedingungen, die Umwandeln des Gases in Essigsäure in einer Fermentationsbrühe erlauben.

2. Verfahren nach Anspruch 1, weiterhin umfassend Freisetzen von Abgasen aus dem Bioreaktor.

3. Verfahren nach Anspruch 1, weiterhin umfassend Abtrennen des Bakteriums von der Fermentationsbrühe.

4. Verfahren nach Anspruch 3, weiterhin umfassend Rückführen des entfernten Bakteriums in den Bioreaktor.

5. Verfahren nach Anspruch 1, wobei in einer Extraktionskammer eine Lösungsmittelphase, welche Essigsäure und Wasser enthält, und eine wässrige Phase, die Nährstoffe enthält, gebildet werden.

6. Verfahren nach Anspruch 5, weiterhin umfassend Abtrennen einer wässrigen Phase, welche Nährstoffe enthält, von der Lösungsmittelphase, welche Lösungsmittel, Essigsäure und Wasser enthält, und Überführen der wässrigen Phase, welche Nährstoffe und Materialien enthält, an den Bioreaktor.

7. Verfahren nach Anspruch 6, weiterhin umfassend Destillieren der Lösungsmittelphase, um Essigsäure und Wasser von dem nicht mit Wasser mischbaren Lösungsmittel abzutrennen.

8. Verfahren nach Anspruch 7, weiterhin umfassend Rückführen des nicht mit Wasser mischbaren Lösungsmittels zur Extraktionskammer.

9. Verfahren nach Anspruch 8, weiterhin umfassend Abtrennen der Essigsäure vom Wasser in der abschließenden Destillationssäule und Rückführen des Wassers in den Bioreaktor.

10. Verfahren nach einem der Ansprüche 1 bis 9, wobei das Gas (i) oder (ii) außerdem Kohlendioxid enthält.

11. Verfahren nach einem der Ansprüche 1 bis 10, wobei das Gas außerdem eines oder mehrere der Gase Stickstoff oder Methan enthält.

12. Verfahren nach einem der Ansprüche 1 bis 11, wobei das Gas durch ein technisches Verfahren hergestellt wird ausgewählt aus der Gruppe bestehend aus Ruß-, Ammoniak-, Methanolherstellung oder Koks- oder Erdölraffination.

13. Verfahren nach einem der Ansprüche 1 bis 12, wobei der Bioreaktor ein kontinuierlich gerührter Tankreaktor, ein immobilisierter mikrobieller Zellreaktor, ein Rieselbettreaktor, ein Blasensäulen-Bioreaktor oder ein Gas-Lift-Reaktor ist.

14. Verfahren nach einem der Ansprüche 1 bis 13, wobei der Bioreaktor bei einem Druck von mehr als einer Atmosphäre (1 x 10⁵ N.m⁻²) gehalten wird.

15. Verfahren nach einem der Ansprüche 1 bis 13, wobei das Verfahren bei einem Druck von bis zu 15 Atmosphären (1,5 x 10⁶ N.m⁻²) durchgeführt wird.

16. Verfahren nach Anspruch 3, wobei das Trennen durch Zentrifugieren, Hohlfasermembranfiltration, Absetzen oder Ultrafiltration erreicht wird.

17. Verfahren nach einem der Ansprüche 1 bis 16, wobei der Bioreaktor weiterhin ein anderes anaerobes acetogenes Bakterium enthält, das aus der Gruppe bestehend aus *Acetobakterium kivui, A. woodii, Butyribakterium methylotrophicum, Clostridium aceticum, C*. *acetobutylicum, C*. *formoaceticum, C. kluyveri, C. ljungdahlii PETC, C. thermoaceticum, C. thermocellum, C. thermohydrosulfuricum, C. thermosaccharolyticum, Eubacterium limosum* und *Peptostreptococcus productus* ausgewählt ist.

18. Verfahren nach Anspruch 1, weiterhin umfassend Inkontaktbringen der Essigsäure mit dolomitischem Kalk und Magnesiumoxid und Trocknen, wobei Calciummagnesiumacetat hergestellt wird.

19. Verfahren nach Anspruch 1, weiterhin umfassend Inkontaktbringen der Essigsäure mit Ätzkali, wodurch Kaliumacetat hergestellt wird.

20. Verfahren nach einem der Ansprüche 1 bis 19, wobei der pH-Wert im Bioreaktor 4,9 beträgt.

21. Verfahren nach Anspruch 1, weiterhin Folgendes umfassend:
(d) kontinuierliches Entfernen eines Teils der Essigsäure enthaltenden Fermentationsbrühe aus dem Bioreaktor;
(e) Inkontaktbringen der entfernten Fermentationsbrühe, die die Essigsäure enthält, mit einem mit Wasser nicht mischbaren Lösungsmittel, das eine Affinität zu Essigsäure besitzt; und
(f) optional Abdestillieren der Essigsäure von dem Lösungsmittel.

22. Verfahren nach Anspruch 21, wobei nach Schritt (e) oder (f) die Essigsäure mit dolomitischem Kalk und Magnesiumoxid in Kontakt gebracht und getrocknet wird, wodurch Calciummagnesiumacetat hergestellt wird.

23. Verfahren nach Anspruch 21, wobei nach Schritt (e) oder (f) die Essigsäure mit Ätzkali in Kontakt gebracht wird, wodurch Kaliumacetat hergestellt wird.

24. Verfahren nach einem der Ansprüche 1 bis 23, wobei der Bioreaktor weiterhin ein Tensid enthält, das den Kohlenmonoxidverbrauch durch das Bakterium erhöht.

25. Biologisch reine Kultur des Mikroorganismus *Clostridium Ijungdahlii* ERI-2, ATCC Nr. 55380.

## Revendications

1. Procédé de production d'acide acétique, comprenant :
(a) la fourniture d'un flux continu de gaz, comprenant :
(i) un gaz comprenant du monoxyde de carbone ;
(ii) un gaz comprenant du monoxyde de carbone et de hydrogène ; ou
(iii) un gaz comprenant de l'hydrogène et du dioxyde de carbone ;
dans un bioréacteur, ledit bioréacteur contenant un milieu nutritif aqueux et la bactérie anaérobie *C. ljungdahlii* ERI-2, ATCC N° 55380 ;
(b) le guidage d'un flux dudit milieu nutritif aqueux dans ledit bioréacteur ; et
(c) la fermentation dudit gaz et dudit milieu nutritif aqueux en utilisant ladite bactérie à un pH inférieur, dans des conditions permettant de convertir le gaz en ledit acide acétique dans un bouillon.

2. Procédé selon la revendication 1, comprenant en outre le dégagement de gaz d'échappement à partir dudit bioréacteur.

3. Procédé selon la revendication 1, comprenant en outre la séparation de ladite bactérie vis-à-vis dudit bouillon.

4. Procédé selon la revendication 3, comprenant en outre le retour de ladite bactérie extraite audit bioréacteur.

5. Procédé selon la revendication 1, dans lequel une phase de solvant, contenant de l'acide acétique et de l'eau, et une phase aqueuse, contenant des substances nutritives, sont formées dans une chambre d'extraction.

6. Procédé selon la revendication 5, comprenant en outre la séparation, vis-à-vis de ladite phase de solvant contenant du solvant, de l'acide acétique et de l'eau, d'une phase aqueuse contenant des substances nutritives, et le passage de ladite phase aqueuse contenant des substances nutritives et des matières dans ledit bioréacteur.

7. Procédé selon la revendication 6, comprenant en outre la distillation de ladite phase de solvant, de manière à séparer l'acide acétique et l'eau dudit solvant non miscible dans l'eau.

8. Procédé selon la revendication 7, comprenant en outre le retour dudit solvant non miscible dans l'eau à ladite chambre d'extraction.

9. Procédé selon la revendication 8, comprenant en outre la séparation dudit acide acétique vis-à-vis de ladite eau, dans une colonne de distillation finale, et le recyclage de ladite eau audit bioréacteur.

10. Procédé selon l'une quelconque des revendications 1 à 9, dans lequel le gaz (i) ou (ii) contient en outre du dioxyde de carbone.

11. Procédé selon l'une quelconque des revendications 1 à 10, dans lequel ledit gaz comprend en outre un ou plusieurs constituants parmi l'azote et le méthane.

12. Procédé selon l'une quelconque des revendications 1 à 11, dans lequel ledit gaz est produit par un procédé industriel, sélectionné dans le groupe composé de la fabrication de noir de carbone, gaz ammoniac, méthanol ou coke ou raffinerie de pétrole.

13. Procédé selon l'une quelconque des revendications 1 à 12, dans lequel ledit bioréacteur est un réacteur continu continûment agité, un bioréacteur à cellules microbiennes immobilisées, un bioréacteur à lit garni cocourant descendant, un bioréacteur à colonne à bulles, ou un bioréacteur à extraction au gaz.

14. Procédé selon l'une quelconque des revendications 1 à 13, dans lequel ledit bioréacteur est maintenu à une pression supérieure à une atmosphère (1x10⁵ N.m⁻²).

15. Procédé selon l'une quelconque des revendications 1 à 13, dans lequel ledit procédé est conduit à une pression allant jusqu'à 15 atmosphères de pression (1,5x10⁶ N.m⁻²).

16. Procédé selon la revendication 3, dans lequel ladite séparation est accomplie par centrifugation, filtration sur membrane à fibres creuses, décantation ou ultrafiltration.

17. Procédé selon l'une quelconque des revendications 1 à 16, dans lequel ledit bioréacteur comprend en outre une autre bactérie acétogène anaérobie, sélectionnée dans le groupe composé de *Acetobacterium kivui, A. woodii, Butyribacterium methylotrophicum. Clostridium aceticum, C. acetobutylicum, C. formoaceticum, C. kluyveri, C. ljungdahlii PETC, C. thermooaceticum, C. thermocellum, C. thermohydrosulfuricum, C. thermosaccharolyticum, Eubacterium limosum, et Peptostreptococcus productus.*

18. Procédé selon la revendication 1, comprenant en outre la mise en contact de l'acide acétique avec de la chaux dolomitique et de l'oxyde de magnésium et le séchage, de manière à produire de l'acétate de calcium-magnésium.

19. Procédé selon la revendication 1, comprenant en outre la mise en contact de l'acide acétique avec de la potasse caustique, de manière à produire de l'acétate de potassium.

20. Procédé selon l'une quelconque des revendications 1 à 19, dans lequel le pH dans le bioréacteur est de 4,9.

21. Procédé selon la revendication 1, comprenant en outre :
(d) l'extraction continue, à partir dudit bioréacteur, d'une partie dudit bouillon contenant l'acide acétique ;
(e) la mise en contact dudit bouillon extrait, contenant l'acide acétique, avec un solvant non miscible dans l'eau, présentant une affinité envers l'acide acétique ; et
(f) en option, la distillation de l'acide acétique à partir dudit solvant.

22. Procédé selon la revendication 21, dans lequel, après l'étape (e) ou (f), l'acide acétique est mis en contact avec de la chaux dolomitique et de l'oxyde de magnésium et séché, de manière à produire de l'acétate de calcium-magnésium.

23. Procédé selon la revendication 21, dans lequel, après l'étape (e) ou (f), l'acide acétique est mis en contact avec de la potasse caustique, de manière à produire de l'acétate de potassium.

24. Procédé selon l'une quelconque des revendications 1 à 23, dans lequel ledit bioréacteur contient en outre un tensio-actif, augmentant la consommation de monoxyde de carbone par ladite bactérie.

25. Culture, bactériologiquement pure, du microorganisme *Clostridium ljungdahlii* ERI-2, ATCC N° 55380.
